# EUROPEAN PATENT SPECIFICATION

(11) **EP 0 681 601 B1**
(45) Date of publication and mention of the grant of the patent: **17.02.1999**
(21) Application number: 95904747.3
(22) Date of filing: 29.11.1994
(51) Int. Cl.: C09J 4/00, C09J 133/06

(54) **WATER-SOLUBLE PRESSURE SENSITIVE ADHESIVE**
WASSERLÖSLICHER, DRUCKEMPFINDLICHER KLEBSTOFF
ADHESIF AUTOCOLLANT SOLUBLE DANS L'EAU

(30) Priority: 29.11.1993 US 158405; 11.03.1994 US 208599; 11.07.1994 US 272827
(43) Date of publication of application: 15.11.1995
(73) Proprietor: ADHESIVES RESEARCH, INC., Glen Rock, PA 17327 (US)
(72) Inventor: ZAJACZKOWSKI, Michael, J., Yoe, PA 17313 (US)
(74) Representative: Burford, Anthony Frederick
(86) International application number: US9413283
(87) International publication number: WO9514746

(56) References cited:
- EP-A- 0 063 037
- DATABASES "CHEMICAL ABSTRACTS" and "REGISTRY" (HOST: STN); abstract 100:52757 and corresponding R.N.'s, Colombus, OH,US. & JP,A,58 108 278 (DAINIPPON PRINTING CO.)

## Description

The present invention is directed to a water-soluble or water-dispersible normally tacky pressure sensitive adhesive.

In papermaking and printing processes the ends of the paper rolls are spliced together to enable the formation of endless paper webs. Splicing of paper within a roll is also undertaken when the paper is cut to remove defective portions. For these purposes, it has been customary to use a repulpable pressure sensitive adhesive-based splicing tape. As various portions of the paper roll or web are cut out and recycled to the paper-making process, it is important for any adhesive which is used in the splice to be readily water-soluble or repulpable (i.e., readily disperses or dissolves in water during a pulping process) so as to not contaminate or discolor the paper which is produced upon being recycled. It is also important for the adhesive splice to be strong enough to resist web failure during the papermaking process or printing process as well as the ability to withstand elevated processing (such as printing) temperatures that may be encountered.

Off-machine paper coating processes also require the use of splicing tapes that exhibit the requisite tack, temperature resistance, and humidity resistance. It would also be desirable for such splicing tapes to exhibit repulpability. However, to date, few commercially acceptable splicing tapes exist that meet the stringent demands of off-machine coaters which subject the tape to excess amounts of water during the coating process as well as elevated temperatures. Also, to date, no commercially-acceptable repulpable splicing tape has been formed that may be satisfactorily employed in off-machine coaters.

Splicing tapes also frequently contribute to a failure in the splice in the paper web as a result of the combination of the speed of the web and the fact that the spliced web is eventually collected in the form of a roll and the splice is caused to conform to the curved surface of the roll. Such prior art splicing tapes employ an adhesive that is sufficiently inelastic such that the splice is incapable of withstanding the rigors encountered during rolling of the web. Such splicing failures contribute to significant inefficiency and expense, as such splice failures must be repaired.

A need also exists for pressure sensitive adhesive labels which can be easily removed from containers or other surfaces to which the adhesive-backed label has been applied. This need exists in particular with respect to containers made from plastic or glass which are intended to be reused. It is accordingly desirable to provide labels which are easily removable by means of water, which labels are most preferably water-soluble or dispersible.

Various attempts have been made to solve such problems as discussed in US-A-3,441,430; US-A-3,556,835; US-A-3,660,147; US-A-4,413,080; US-A-4,413,082; US-A-4,992,501; US-A-5,084,348; US-A-5,094,912; US-A-5,098,962; US-A-5,102,733; US-A-5,125,995; US-A-5,141,810; US-A-5,183,841; US-A-5,196,504; and WO-A-93/06184. Such alternative solutions require, for example, the use of added tackifying agents or result in adhesives which are soluble in aqueous alkaline solutions. However, the prior art reliance upon water-soluble tackifying agents to impart the required water-solubility for the pressure sensitive adhesive (especially in repulpable adhesives) results in several disadvantages. The prior art relies on the ability of the tackifier to be removed from the adhesive composition in the presence of water (with the intended result that the composition cannot serve as an adhesive due to loss of tack). However, under conditions of high humidity the tackifier may migrate from the adhesive to the adjacent substrate (such as the paper roll). The paper may, as a result, be spotted or discolored by the migrating tackifier. In order to avoid such a consequence, repulpable adhesive tapes are generally sealed in bags to protect against excess humidity. This packaging requirement is costly and burdensome, and as yet unavoidable.

An additional disadvantage that exists with regard to tackified repulpable pressure sensitive adhesives is the fact that the tackified base polymer is itself non-water soluble. As a result the potential for contamination exists due to the presence of the non-water soluble adhesive once the tackifier is solubilized.

Thus, a need exists for a pressure sensitive adhesive which exhibits enhanced water-soluble or water-dispersible characteristics without the need for added tackifying agents and whose water-solubility may be tailored for specific applications. It is also desirable to provide a repulpable water-soluble or water-dispersible pressure sensitive adhesive which is resistant to loss of adhesive properties upon exposure to conditions of high humidity and high temperature, and which does not serve as a source of contamination or discoloration. It is also desirable to provide a high performance pressure sensitive adhesive that is also water-soluble or water-dispersible.

JP-A-58108278 discloses an ionizing radiation curing-type pressure-sensitive composition comprising:
(A) 60-95 wt% of a polymer prepared from a C₂-C₁₄ alkyl acrylate (e.g. butyl acrylate, 2-ethylhexyl acrylate) or a copolymer prepared from (i) 60-95 wt% of a said acrylate, (ii) 5-40 wt% of a monomer selected from vinyl acetate, (meth)acrylic acid, C₂-C₁₄ methacrylic esters and acrylamide, having a molecular weight of 5,000-500,000 and
(B) 5-40 wt% of a second polymer prepared from an alkoxylated acrylate monomer having C₁-C₈ alkyl and C₂-C₄ alkylene groups and a molecular weight 130-2,000 and optionally
(C) at most 35 wt% (based on the combined weight of components A and B) of an acryloyl group-containing monomer (e.g. methyl acrylate).
An exemplified composition comprises 100 parts of a copolymer prepared from 75 wt% butyl acrylate, 2 wt% acrylic acid, 3 wt% diacetone acrylamide & 20 wt% vinyl acetate and 20 parts CH₃O(CH(CH₃)CH₂O)₂OCCH=CH₂. The compositions have high cohesive ability, stickiness and solvent resistance.

It is one object of the present invention to provide a water-soluble or water-dispersible pressure sensitive adhesive.

It is further an object of the present invention to provide a water-soluble or water-dispersible pressure sensitive adhesive which may be used in the absence of tackifiers or plasticizers.

It is still further an object of the present invention to provide a water-soluble pressure sensitive adhesive which exhibits high performance adhesive properties as well as humidity and temperature resistance and water dispersibility.

It is also an object of the present invention to provide a repulpable pressure sensitive adhesive.

It is further an object of the present invention to provide adhesive-coated backing materials such as sheets or tapes coated with such water-soluble or water-dispersible adhesives.

In accordance with the present invention, there is thus provided a water-soluble or water-dispersible normally-tacky pressure sensitive adhesive comprised of a graft copolymer comprised of one or more water-soluble base monomers A and a water-soluble or water-dispersible macromer C, and optionally one or more B monomers copolymerized with said A monomer, wherein said base monomer A comprises a vinyl monomer capable of forming a hydrophilic polymer and having a T_{g} <20°C, said optional monomer B being capable of forming a hydrophilic or hydrophobic polymer, and said C macromer forming polymeric sidechains of said graft copolymer, with the provisos that when said C macromer is present in an amount of at least 45 percent by weight, then at least 5 percent by weight of a B monomer having a T_{g} >20°C is present, and when said C macromer is present in an amount of 35 percent by weight or less, then at least 5 percent by weight of a B monomer having a T_{g} <0°C is present, and wherein any B monomer present, if hydrophobic, is present in an amount of 25 percent by weight or less, based on the total weight of the components A, B and C, said adhesive exhibiting a tackifier or plasticizer-free Polyken probe back value of at least 300 g/cm² as determined by ASTM 02979, a peel adhesion value of at least 20 oz/in (220 g/cm) as determined by PSTC 1, and a shear holding value of greater than 5 minutes as determined at 500 grams according to PSTC 7.

In accordance with a preferred embodiment of the present invention, there is also provided a water-soluble or water-dispersible normally tacky pressure sensitive adhesive comprised of a graft copolymer comprised of one or more water-soluble base monomers A and a water-soluble or water-dispersible macromer C, and optionally one or more B monomers copolymerizable with said A monomer, wherein said base monomer A comprises a vinyl monomer capable of forming a hydrophilic polymer and having a T_{g} <20°C., said optional monomer B is capable of forming a hydrophilic or hydrophobic polymer, and said macromer C forming polymeric sidechains of said graft copolymer and comprising a hydrophilic macromer represented by the formula:

X-(Y)ₚ-(O-CₘH₂ₘ)ₙ-R

wherein X is a moiety copolymerizable with monomers A and B or capable of attachment to polymerized monomers A and B, Y is a divalent linking group, R is a terminal group; and in which m is an integer of from 2 to 6, n is an integer of from 5 to 300, and p is 0 or 1.

In accordance with the present invention, there is also provided an adhesive-coated tape or sheet material having a portion of at least one surface thereof coated with the adhesive of the present invention.

The normally-tacky pressure sensitive adhesive of the present invention comprises a water-soluble or water-dispersible graft copolymer of at least one water-soluble base monomer and a hydrophilic macromer. The term "tacky" is intended to mean that the polymer is sticky by nature and exhibits adhesion upon contact with a surface.

The water-soluble base monomer A comprises a vinyl monomer capable of forming a hydrophilic polymer and having a T_{g} <20°C. In general, such monomers may comprise hydroxy(C₁-C₅)alkyl acrylates, hydroxy(C₁-C₅)alkyl methacrylates, dihydroxy(C₁-C₅)alkyl acrylates, or dihydroxy(C₁-C₅)alkyl methacrylates. Exemplary water-soluble base monomers include but are not limited to hydroxyethyl acrylate, hydroxypropyl acrylate, hydroxybutyl acrylate, hydroxybutyl methacrylate, as well as alkyl vinyl ethers and hydroxy alkyl vinyl ethers (wherein the alkyl group has up to 5 carbon atoms). One or more of the water-soluble A monomers may be employed.

The macromer C forms polymeric sidechains of the graft copolymer. The macromer C is hydrophilic by nature (i.e., the macromer is water-soluble or water-dispersible).

The macromer may be represented by the formula X-(Y)ₚ-Z-R wherein X is a moiety copolymerizable with monomers A and B or, in the alternative, capable of attachment to polymerized monomers A and B, Y is a divalent linking group, Z is a water-soluble or water-dispersible homo- or polymeric moiety essentially unreactive at copolymerization conditions, R is a terminal group, and p is 0 or 1.

More specifically, the X moiety is an unsaturated polymerizable moiety the composition of which is not critical. The X moiety may be, for example, when intended to be copolymerizable with monomers A and B, simply a vinyl group of the formula CHR=CR¹- where R is hydrogen or COOH and R¹ is hydrogen or alkyl such as methyl. Other exemplary X moieties include but are not limited to methacryloyl, maleoyl, itaconoyl, crotonoyl, unsaturated urethane moiety, methacrylamido and moieties of the formula CH₂=CHCH₂O-.

The X moiety may comprise an amine or alcohol moiety (such as a monohydroxyl or monoamine moiety) which permits attachment of the macromer to a suitable functionality on previously-polymerized monomers A and B. For instance, the hydroxyl moiety can serve as a terminal reactive group by reaction with suitable moieties on the polymer backbone resulting from the use of monomers such as isocyanate-substituted (meth)acrylic acid or (meth)acrylic acid anhydride.

A preferred Y divalent linking group is 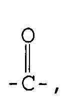 or a linking group which incorporates such a moiety.

Additional Y linking groups which may be employed in connection with the present invention include but are not limited to the following moieties: 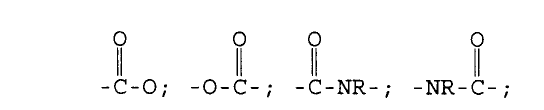 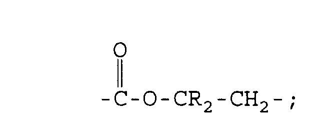 -CH₂-O-CR₂-CH₂-; -O-CR₂-CH₂-O-CR₂-CH₂-; -OCH₂CH₂-O-CR₂-CH₂-; and 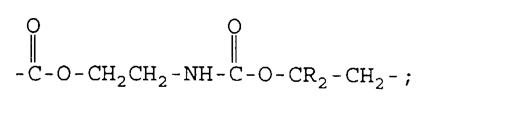 where R is hydrogen, alkyl or phenyl. Obviously, the presence of the Y linking group is optional in the event the moiety includes a functionality which enables the Z moiety to react with the X moiety. As the incorporation of macromolecular moieties in copolymers is well understood by those skilled in the art, the choice of a suitable X and Y moiety for use in the present invention may be readily made upon practice of the present invention. See, for example, the discussion in US-A-3,786,116; US-A-3,832,423; US-A-3,842,058; US-A-3,842,059; US-A-3,842,146; and US-A-4,554,324.

The Z moiety is preferably comprised solely of one or more hydrophilic monomer radicals to ensure that the resulting macromer is water-soluble or water-dispersible. However, the Z moiety may also be a copolymer of hydrophilic and hydrophobic monomers, with any copolymerized hydrophobic portion being present in an amount insufficient to render the resulting macromer water-insoluble or non-water-dispersible. Desirably, any non-hydrophilic portion employed in such a copolymer is present in an amount of less than 50 percent by weight based on the weight of the macromer, and preferably less than 30 percent by weight.

The Z moiety is more preferably selected from (but not limited to) a polyalkylene oxide radical such as a polypropylene or polyethylene oxide radical, a polyethyloxazoline radical such as a radical of poly(2-ethyl-2-oxazoline), polyacrylic acid radical, polyvinyl alcohol radical, polyvinylpyrrolidone radical, polyvinyl caprolactam radical, polymethylvinyl ether radical or mixtures thereof. Exemplary C macromers formed from such radicals include but are not limited to ethoxylated or propoxylated hydroxy(C₁-C₅)alkyl (C₁-C₅ alk)acrylate and polymethylvinyl ether mono(meth)acrylate. The molecular weight of the macromer used in the present invention is not critical but will generally range from 300 to 50,000, and preferably from 300 to 3,000.

The hydrophilic macromer C is most preferably represented by the formula:

X-Y-(O-CₘH₂ₘ)ₙ-R

wherein X and Y are as defined above and R represents a terminal group; and in which m is an integer of from 2 to 6 and n is an integer of from 5 to 300. More specifically, macromer C is advantageously an ethoxylated or propoxylated hydroxy(C₁-C₅)alkyl (C₁-C₅ alk)acrylate represented by the formula: 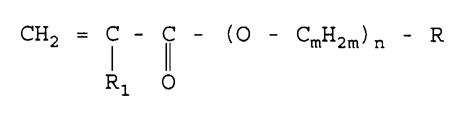 wherein R¹ is hydrogen or C₁₋₅ alkyl and R is a terminal group. Preferably, m is 2 or 3 and n is 5 to 30, and R is OH or C₁₋₅ alkyl.

The macromer C may employ a variety of terminal groups R. While the terminal group may typically be OH or C₁₋₅ alkyl, it may be desirable to select a terminal group based on the functional character of the terminal group. For instance, suitable terminal groups include but are not limited to (1) acid/ionic groups such as carboxyl, phosphate or sulfate groups, (2) hydrophobic groups such as C₁-C₅ alkyl, phenyl or substituted phenyl, and (3) hydrophilic groups such as hydroxyl or amine groups.

Depending upon the terminal group employed, ionic end groups may be used to provide pH-dependent solubility characteristics for the copolymer. Hydrophobic terminal groups may be used to reduce the water solubility of the copolymer.

Other physical properties or characteristics of the copolymer may be modified by selection of suitable terminal groups. Ionic terminal groups may be used to provide a desired degree of cross-linking; for example, by neutralizing acid moieties with metal hydroxides. High temperature performance may be enhanced by incorporating an acid functionality in conjunction with a ditertiary amine. Aqueous solution viscosities may be influenced by the presence of ionic terminal groups.

As discussed above, one or more polymerizable B monomers may be incorporated in the copolymer which B monomer(s) is copolymerizable with the A monomer. Such additional B monomer(s) may be either hydrophilic or hydrophobic. In the event that a reduced degree of water solubility of the adhesive is acceptable (such as for non-repulp applications), it may be advantageous to incorporate a hydrophobic B monomer in an amount that renders the polymer composition either water-insoluble or reduces the extent or degree of water solubility of the polymer. For instance, the presence of from 30-40 percent by weight of a hydrophobic B monomer in the composition will render the adhesive non-soluble but water-dispersible or, in the alternative, insoluble (depending upon the amount of the hydrophobic B monomer employed). For applications where it is desirable to enhance the water-solubility or water-dispersibility of the adhesive, it is preferable for the B monomer, if hydrophobic, to be present in an amount of 25 percent by weight or less, and most preferably 20 percent by weight or less. Generally, in a repulp application, the B monomer will be present in an amount of 15 percent by weight or less.

Exemplary optional B monomers include water-soluble vinyl monomers having at least one nitrogen atom. Such monomers (each of which exhibit a T_{g} of >20°C.) include but are not limited to N-mono-substituted acrylamides such as acrylamide, methacrylamide, N-methylacrylamide, N-ethylacrylamide, N-methylolacrylamide, N-hydroxyethylacrylamide, and diacetone acrylamide; N,N-disubstituted acrylamides such as N,N-dimethylacrylamide, N,N-diethylacrylamide, N-ethyl-N-aminoethyl acrylamide, N-ethyl-N-hydroxyethylacrylamide, N,N-dimethylolacrylamide, and N,N-dihydroxyethylacrylamide.

Other optional B monomers may include, for example, various vinyl monomers such as acrylic and methacrylic acids, methoxyethyl acrylate or methacrylate, ethoxyethyl acrylate or methacrylate, methyl acrylate or methacrylate, ethyl acrylate or methacrylate, propyl acrylate or methacrylate, glycerol acrylate or methacrylate, hydroxyethyl methacrylate, hydroxypropyl methacrylate, vinyl pyrrolidone and vinyl caprolactam. Monomeric acrylic or methacrylic acid esters of a non-tertiary alcohol having from 4-12 carbon atoms on average, and preferably from 4-8 carbon atoms, such as n-butyl acrylate or methacrylate, are also suitable B monomers. One or more B monomers may be employed.

In one preferred embodiment, the adhesive of the invention comprises an hydroxy(C₁-C₅)alkyl acrylate as A monomer copolymerized with acrylamide, butyl acrylate, vinyl pyrrolidone and acrylic acid as B monomers, and a water-soluble or water-dispersible macromer.

Preferably, said A monomer is present in an amount of from 25 to 70 percent by weight, said optional B monomer is present in an amount of from 0 to 40 percent by weight, and said C macromer is present in an amount of from 10 to 65 percent by weight, and preferably 30 to 60 percent by weight, all amounts based on the total weight of the components A, B and C in the composition. If both hydrophilic and hydrophobic B monomers are present, the amount of the hydrophobic B monomer present is preferably insufficient to exceed 25 percent by weight of the total weight of the monomers A, B and C.

Preferably, the composition contains at least 35 percent by weight of the A monomer and at least 30 percent by weight of the C macromer. Most preferably, at least 40 percent by weight of the A monomer is present. Most preferably, at least 35 percent by weight of the C macromer is present.

When the C macromer is present in an amount of at least 45 percent by weight, then at least 5 percent by weight of a B monomer having a T_{g} of >20°C. is present, and when the C macromer is present in an amount of 35 percent by weight or less, then at least 5 percent by weight of a B monomer having a T_{g} of <0°C. is present in order to ensure that the composition of the present invention exhibits satisfactory adhesive properties of probe tack, peel and shear.

If less than 30 percent by weight of the C macromer is employed, it may be necessary to incorporate a water-soluble tackifier or plasticizer into the composition to provide sufficient tack. Exemplary tackifiers include polyethylene glycol, polypropylene glycol, and suitable polyoxyethylene-based compounds. Suitable polyoxyethylene-based tackifiers are disclosed at column 6 of US-A-4,413,080. Such tackifiers, if present, may be employed in an amount of up to 50 percent by weight, based on the total weight of the composition. While the use of a tackifier or plasticizer in repulp applications is not generally seen to be an advantage, it is possible that the tackifier or plasticizer may actually be used with advantage in non-repulp applications, and may accordingly be present in the composition as found appropriate.

Most preferably, in order to provide a "tackifier-free" or "plasticizer-free" adhesive for use in repulp applications, the weight ratio of C macromer to total weight of monomers A and B ranges from 35:65 to 65:35, with it being further preferred that the C macromer be present in a weight ratio of 40:60 to 50:50 based on the weight of C macromer to total weight of A and B monomers.

The weight average molecular weight of the resulting polymer is preferably at least 18,000, and may be as high as 100,000-200,000, with the upper limit of molecular weight being that which detracts from the desired water-solubility of the polymer.

By way of advantage, the repulpable pressure sensitive adhesive of the present invention enables the use of a water-soluble tackifier or plasticizer to be avoided (thus avoiding potentially-damaging tackifier migration problems). The C macromer itself serves to "tackify" the adhesive composition while avoiding problems associated with migration of the tackifying component as in the prior art. This provides the further advantage that such adhesives, while water soluble, do not need to be protected from excess humidity (such as by bagging) as the adhesive is surprisingly humidity-resistant. The lack of a need to protect such adhesives from the ambient environment results in less waste, greater economic efficiency, and no need for special handling.

A still additional advantage is that the adhesive composition of the present invention employs a water-soluble base polymer in contrast to the water-insoluble base polymer generally used in prior art repulpable compositions. The pressure sensitive adhesive of the present invention is accordingly substantially if not completely water-soluble or dispersible, a result not heretofore accomplished by the prior art adhesive compositions. Little, if any, adhesive residue remains to serve as a potential contaminant upon contact of the adhesive with excess amounts of water.

As noted above, the copolymer composition of the present invention may be prepared by any conventional polymerization technique, including (1) free radical initiated copolymerization of components A and C and optionally B in the presence of a solvent, and (2) attachment of the macromer C graft to a preformed backbone polymer formed from copolymerized monomer A optionally copolymerized with monomer B via reaction with a suitable functional group on the backbone polymer subsequent to formation of same. Suitable copolymerization temperatures range from 20°C. to 150°C. for periods of time of from 2 to 24 hours until the desired degree of conversion occurs. Upon completion of the copolymerization process, the solvent is removed and a tacky copolymer results having acceptable adhesive properties. If desired, a suitable cross-linking agent may be employed to increase the molecular weight of the adhesive if desired.

The adhesive of the present invention may be used in association with a variety of body members to provide an adhesive assembly. For example, the body member may be in the form of a backing material coated on at least one side thereof with the adhesive to provide an adhesive-backed sheet film or tape. Exemplary backing materials used in the production of such a product include but are not limited to flexible and inflexible backing materials conventionally employed in the area of pressure sensitive adhesives, such as creped paper, kraft paper, fabrics (knits, non-wovens, wovens), foil and synthetic polymer films such as polyethylene, polypropylene, polyvinyl chloride, poly(ethylene terephthalate) and cellulose acetate, as well as glass, ceramics, metallized polymer films and other compatible sheet or tape materials.

Preferably, in order to enable the adhesive assembly to exhibit "repulpable" properties, a backing material is employed which is water-dispersible. In such an instance, the backing material is generally comprised of a paper layer, such as kraft paper or tissue paper, although any water-dispersible backing layer may be employed. For instance, water-soluble polymeric materials such as polyvinyl alcohol can also be used as a backing layer.

The body member (e.g., in sheet form) may be coated in any conventional manner with the adhesive composition of the present invention, such as by roll coating, spray coating, extrusion coating, co-extrusion coating, and hot melt coating by use of conventional coating devices. When appropriate, the adhesive of the present invention may be applied as a solution to at least one surface of the body member and the solvent subsequently removed to leave a tacky adhesive residue on the body member. The adhesive may be applied to the body member either in the form of a continuous layer or in discontinuous form.

The resulting pressure sensitive adhesive-backed body member may take many forms, such as tapes, patches, strips, and labels, with the choice and form ultimately being determined by the end use contemplated.

The pressure sensitive adhesive of the present invention may be used in a wide variety of commercial applications. An adhesive tape formed in accordance with the present invention may be used as a splicing tape in paper making, printing and off-machine coating processes while providing advantages not provided by other commercially-available adhesive tapes. An adhesive tape produced according to the present invention provides a combination of high performance characteristics such as tack, water and temperature resistance in combination with water-solubility and water-dispersibility not heretofore provided by present commercially-available alternatives. In contrast to prior art adhesive tapes, the adhesive of the present invention does not sacrifice performance to provide water-soluble or water-dispersible characteristics.

Advantageously, splicing tape produced in accordance with the present invention is also resistant to splicing failures during formation of rolls of spliced paper webs. This resistance to splice failure is believed due to the greater ability of the pressure sensitive adhesive to withstand the rigors of the web rolling process due to its rubbery nature due to the low T_{g} of the adhesive.

The pressure sensitive adhesive of the present invention exhibits a tackifier-free Polyken probe tack value of at least 300 g/cm² and a peel adhesion value of at least 20 oz/in (220 g/cm). Advantageously, the Polyken probe tack value of the adhesive of the present invention will range from 400 to 1500 g/cm² as determined by ASTM D2979, and the peel adhesion value of the adhesive will range from 30 to 100 oz/in (330-1120 g/cm) as determined by PSTC 1. The adhesive of the present invention exhibits a shear holding value of greater than 5 minutes (and preferably greater than 10 minutes) as determined at 500 grams according to PSTC 7.

The invention will be discussed in conjunction with the following examples, which are merely illustrative of the present invention and not intended to in any way limit the scope of the invention. The following Examples demonstrate the use of a water-soluble pressure sensitive adhesive which exhibits desirable characteristics such as peel, tack, shear and water solubility in the production of a repulpable pressure sensitive adhesive tape.

### EXAMPLE 1

180.13 grams of ethyl acetate and 120.09 grams of isopropyl alcohol (as solvents) were charged to a 1-liter reaction vessel. To the charged material, 18.33% of the monomers identified below were added. Under a nitrogen atmosphere, the batch was heated to 71-77°C and 1.27 grams of VAZO™-52 (polymerization initiator) were added. The reactants were allowed to polymerize for 20 minutes to produce a seed polymer capable of solvating the remaining reactants. Single stage polymerization was found to yield a non-processable gel, which result is avoided by the two-step polymerization using a seed reaction. The remaining 81.67% of the monomer mix along with 0.75 grams of benzoyl peroxide were added to the reaction mix over 2 hours while maintaining a reaction temperature of 71-77°C. The reactants were polymerized until all monomers were consumed. The reactor feed mix consisted of the following components:

| Monomers | Amount (Grams) |
|---|---|
| HEMA-10 (macromer) | 114.09 |
| HEMA-5 (macromer) | 26.34 |
| Hydroxy Ethyl Acrylate (A monomer) | 83.88 |
| Hydroxy Propyl Acrylate (A monomer) | 100.67 |
| Acrylamide (B monomer) | 9.06 |

| Solvents | Amount (Grams) |
|---|---|
| Ethyl acetate | 180.13 |
| Isopropyl alcohol | 120.09 |
| Note: HEMA-5,10 are 5 and 10 mole ethoxylates of hydroxy ethyl methacrylate (produced by BIMAX, INC.) VAZO™-52: Dupont trade name for free radical initiator 2,2'-azobis (2,4-dimethylpentanenitrile). | |

The thus-produced "tackifier-free" water-soluble adhesive composition was coated onto a conventional release sheet and dried to remove the solvent and provide a layer of pressure sensitive adhesive of a weight of 1 oz/yd² (35 g/m²⁾. Tissue paper was then applied over the adhesive surface after which a second layer of adhesive having the same coated weight was applied to the reverse side of the tissue by contacting the tissue with adhesive on another release sheet. A double faced tape was thus produced having a water-soluble pressure sensitive adhesive thereon which tape readily dissolves or disperses in water.

### EXAMPLE 2

The procedure of Example 1 was repeated using the following reactor feed components to produce a tackifier-free water-soluble pressure sensitive adhesive:

| Monomers | Amount (Grams) |
|---|---|
| HEMA-10 (macromer) | 134.22 |
| Hydroxy Ethyl Acrylate (A monomer) | 97.31 |
| Acrylamide (B monomer) | 9.06 |
| Butyl Acrylate (B monomer) | 57.04 |
| Vinyl Pyrrolidone (B monomer) | 20.13 |
| Acrylic Acid (B monomer) | 13.42 |

| Solvents | Amount (Grams) |
|---|---|
| Ethyl acetate | 150.11 |
| Isopropyl alcohol | 150.11 |

### EXAMPLE 3

The procedure of Example 1 was repeated using the following reactor feed components to produce a tackifier-free water-soluble pressure sensitive adhesive:

| Monomers | Amount (Grams) |
|---|---|
| HEMA-10 (macromer) | 113.50 |
| Hydroxy Ethyl Acrylate (A monomer) | 107.38 |
| Acrylamide (B monomer) | 6.56 |
| Butyl Acrylate (B monomer) | 54.73 |
| Vinyl Pyrrolidone (B monomer) | 40.25 |
| Acrylic Acid (B monomer) | 13.12 |

| Solvents | Amount (Grams) |
|---|---|
| Ethyl acetate | 180.13 |
| Isopropyl alcohol | 120.09 |

### EXAMPLE 4

The procedure of Example 1 was repeated using the following reactor feed components to produce a tackifier-free water-soluble pressure sensitive adhesive:

| Monomers | Amount (Grams) |
|---|---|
| HEMA-10 (macromer) | 134.22 |
| Hydroxy Ethyl Acrylate (A monomer) | 97.31 |
| Hydroxy Propyl Acrylate (A monomer) | 40.27 |
| Acrylamide (B monomer) | 10.06 |
| Butyl Acrylate (B monomer) | 23.56 |
| Vinyl Pyrrolidone (B monomer) | 10.06 |
| Acrylic Acid (B monomer) | 10.06 |

| Solvents | Amount (Grams) |
|---|---|
| Ethyl acetate | 160.11 |
| Isopropyl alcohol | 140.11 |

### EXAMPLE 5

The procedure of Example 1 was repeated using the following reactor feed components to produce a tackifier-free water-soluble pressure sensitive adhesive:

| Monomers | Amount (Grams) |
|---|---|
| HEMA-10 (macromer) | 268.43 |
| Hydroxy Ethyl Acrylate (A monomer) | 181.20 |
| Hydroxy Propyl Acrylate (A monomer) | 181.20 |
| Acrylamide (B monomer) | 20.13 |
| Acrylic Acid (B monomer) | 20.13 |

| Solvents | Amount (Grams) |
|---|---|
| Ethyl Acetate | 345.25 |
| Isopropyl Alcohol | 255.19 |

### EXAMPLE 6

94.5 grams of ethyl acetate and 130.5 grams of isopropyl alcohol (or solvents) were charged to a 1-liter reaction vessel. To the charge, 18.33% of the monomers identified below were added. Under a nitrogen atmosphere, the batch was heated to 71-77°C and 0.94 grams of VAZO™-52 (polymerization initiator) were added. The reactants were allowed to polymerize for 20 minutes. The remaining 81.67% of the monomer mix along with 0.50 grams of benzoyl peroxide were added to the reaction mix over 1 hour while maintaining a reaction temperature of 71-77°C. The reactants were polymerized until all monomers were consumed (123.75 grams of isopropyl alcohol were added to reduce viscosity). The monomer feed consisted of the following:

| Monomers | % of Monomers | Amount (Grams) |
|---|---|---|
| HEMA-10 (macromer) | 38.13 | 90.00 |
| Poly(2-Ethyl-2-Oxazoline) macromer | 4.66 | 11.00 |
| Hydroxy Ethyl Acrylate (A monomer) | 23.83 | 56.25 |
| Hydroxy Propyl Acrylate (A monomer) | 10.97 | 25.88 |
| Vinyl Pyrrolidone (B monomer) | 4.77 | 11.25 |
| Vinyl Caprolactam (B monomer) | 4.77 | 11.25 |
| Butyl Acrylate (B monomer) | 12.87 | 30.37 |

The presence of the poly(2-ethyl-2-oxazoline) macromer (5000 mw) enhanced both the cohesive strength and the high temperature performance of the tackifier-free water-soluble pressure sensitive adhesive.

### EXAMPLE 7

The procedure of Example 6 was repeated with the exception that the 2-ethyl-2-oxazoline macromer was not employed:

| Solvents | Amount (Grams) |
|---|---|
| Ethyl Acetate | 189.00 |
| Isopropyl Alcohol | 261.00 |

| Monomers | % of Monomers | Amount (Grams) |
|---|---|---|
| HEMA-10 (macromer) | 40.00 | 179.98 |
| Hydroxy Ethyl Acrylate (A monomer) | 25.00 | 12.50 |
| Hydroxy Propyl Acrylate (A monomer) | 11.50 | 52.29 |
| Vinyl Pyrrolidone (B monomer) | 5.00 | 22.55 |
| Vinyl Caprolactam (B monomer) | 5.00 | 22.55 |
| Butyl Acrylate (B monomer) | 13.50 | 60.76 |

### EXAMPLE 8

Ethyl acetate (25 grams) and isopropyl alcohol (75 grams) (as solvents) were charged to a reaction vessel. To the charge, 19.86% of the monomers identified below were added. Under a nitrogen atmosphere, the batch was heated to 70-73°C and 0.94 grams of VAZO™-52 (polymerization initiator) were added together with 100 grams of additional solvent mixture. The reactants were allowed to polymerize for 20 minutes. The remaining 80.14% of the monomer mix along with benzoyl peroxide catalyst were added to the reaction mix over 1 hour while maintaining a reaction temperature of 70-73°C. The reactants were polymerized until all monomers were consumed. The monomer feed consisted of the following:

| Monomers | % of Monomers | Amount (Grams) |
|---|---|---|
| HEMA-10 (macromer) | 40.00 | 90.00 |
| HEMA-5 | 5.00 | 11.00 |
| Hydroxy Ethyl Acrylate (A monomer) | 22.75 | 56.25 |
| Hydroxy Propyl Acrylate (A monomer) | 8.75 | 25.88 |
| Vinyl Pyrrolidone (B monomer) | 3.50 | 11.25 |
| Vinyl Caprolactam (B monomer) | 3.50 | 11.25 |
| Butyl Acrylate (B monomer) | 15.00 | 30.37 |
| Acrylic acid (B monomer) | 1.50 | |

Upon completion of the reaction, the reaction product is cooled and discharged from the reactor. Before coating on a backing material, 0.15% a cross-linking agent (an aziridine compound such as Hoechst Celanese XAMA-7) is added to the product to increase the temperature resistance of the adhesive.

## Claims

1. A pressure sensitive adhesive comprised of a water-soluble or water dispersible normally-tacky graft copolymer comprised of one or more water soluble base monomers A comprising a vinyl monomer capable of forming a hydrophilic polymer and having a T_{g} <20°C., a water soluble or water dispersible macromer C forming polymeric sidechains of said graft copolymer, and, optionally, copolymerized with said A monomer one or more B monomers capable of forming a hydrophilic or hydrophobic polymer, with the provisos that: when said C macromer is present in an amount of at least 45 percent by weight, then at least 5 percent by weight of a B monomer having a T_{g} of >20°C. is present, when said C macromer is present in an amount of 35 percent by weight or less, then at least 5 percent by weight of a B monomer having a T_{g} of <0°C. is present, and any hydrophobic B monomer present is present in an amount of 25 percent by weight or less, each said amounts being based on the total weight of the components A, B and C, said adhesive exhibiting a tackifier or plasticizer-free Polyken probe tack value of at least 300 g/cm² as determined by ASTM D2979, a peel adhesion value of at least 220 g/cm (20 oz/in) as determined by PSTC 1, and a shear holding value of greater than 5 minutes as determined at 500 grams according to PSTC 7.

2. An adhesive of Claim 1, wherein said A monomer is present in an amount of from 25 to 70 percent by weight, said B monomer is present in an amount of from 0 to 40 percent by weight, and said C macromer is present in an amount of from 10 to 65 percent by weight, based on the total weight of the components A, B and C.

3. An adhesive of Claim 2, wherein said C macromer is present in an amount of at least 35 percent by weight.

4. An adhesive of any one of the preceding claims wherein an hydrophobic B monomer is present in an amount of up to 15 percent by weight, based on the total weight of components A, B and C.

5. An adhesive of any one of the preceding claims, wherein the weight ratio of C macromer to monomers A and B present in said adhesive ranges from 35:65 to 65:35.

6. An adhesive of Claim 5, wherein the weight ratio of C macromer to monomers A and B present in said adhesive ranges from 40:60 to 50:50.

7. An adhesive of any one of the preceding claims, wherein said A monomer is selected from hydroxy(C₁-C₅)alkyl acrylates, hydroxy(C₁-C₅)alkyl methacrylates, dihydroxy(C₁-C₅)alkyl acrylates, dihydroxy(C₁-C₅)alkyl methacrylates and mixtures thereof.

8. An adhesive of any one of the preceding claims, wherein said B monomer which has a T_{g} of >20°C. and is hydrophilic.

9. An adhesive of Claim 8, wherein said B monomer is a water-soluble vinyl monomer having at least one nitrogen atom.

10. An adhesive of Claim 9, wherein said B monomer is selected from N-mono-substituted acrylamides; N,N-disubstituted acrylamides and mixtures thereof.

11. An adhesive of any one of the preceding claims, comprising B monomer which is a vinyl monomer selected from acrylic and methacrylic acids, methoxyethyl acrylate or methacrylate, ethoxyethyl acrylate or methacrylate, methyl acrylate or methacrylate, ethyl acrylate or methacrylate, propyl acrylate or methacrylate, n-butyl acrylate or methacrylate, glycerol acrylate or methacrylate, hydroxyethyl methacrylate, hydroxypropyl methacrylate, vinyl pyrrolidone, vinyl caprolactam and mixtures thereof.

12. An adhesive of any one of the preceding claims, comprising a B monomer which comprises a monomeric acrylic or methacrylic acid ester of a non-tertiary alcohol having from 4-12 carbon atoms on average.

13. An adhesive of any one of the preceding claims, wherein said macromer is defined by the formula X-(Y)ₚ-Z-R, wherein X is a moiety copolymerizable with monomers A and B or capable of attachment to copolymerized monomers A and B, Y is a divalent linking group, Z is a water-soluble or water-dispersible homo- or copolymeric moiety, R is a terminal group, and p is 0 or 1.

14. An adhesive of Claim 13, wherein X is a (meth)acrylate moiety.

15. An adhesive of Claim 13 or Claim 14, wherein Z is selected from a polyalkylene oxide radical, a polyethyloxazoline radical, a polyacrylic acid radical, a polyvinyl alcohol radical, a polyvinylpyrrolidone radical, a polyvinylcaprolactam radical and a polymethylvinyl ether radical.

16. An adhesive of Claim 15, wherein said macromer is defined by the formula:
X-(Y)ₚ-(O-CₘH₂ₘ)ₙ-R
wherein X is a moiety copolymerizable with monomers A and B or capable of attachment to copolymerized monomers A and B, Y is a divalent linking group, R is a terminal group, m is an integer of from 2 to 6, n is an integer of from 5 to 300, and p is 0 or 1.

17. An adhesive of Claim 16, wherein the macromer is defined by the formula 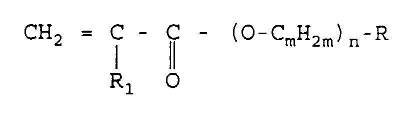 wherein R₁ is hydrogen or C₁₋₅ alkyl and R is a terminal group.

18. An adhesive of Claim 15, wherein m is 2 or 3 and n is an integer of from 5 to 30.

19. The adhesive of any one of Claims 13 to 18 wherein R is OH or C₁₋₅ alkyl.

20. An adhesive of Claim 18 or Claim 19, wherein said macromer is selected from ethoxylated hydroxyethyl (meth)acrylate and ethoxylated hydroxypropyl (meth)acrylate.

21. An adhesive of Claim 15, wherein said macromer is selected from ethoxylated or propoxylated hydroxy(C₁-C₅)-alkyl (C₁-C₅ alk)acrylate and polymethylvinyl ether mono (meth)acrylate.

22. An adhesive of Claim 13, wherein said copolymer comprises an hydroxy(C₁-C₅)alkyl acrylate as A monomer copolymerized with acrylamide, butyl acrylate, vinyl pyrrolidone and acrylic acid as B monomers, and a water-soluble or water-dispersible macromer.

23. An adhesive of any one of Claims 1 to 22, which incorporates a cross-linking agent.

24. An adhesive of Claim 23, wherein the cross-linking agent is added to the preformed graft copolymer.

25. An adhesive of Claim 23 or Claim 24, wherein the cross-linking agent is an aziridine compound.

26. An adhesive of Claim 23, wherein the graft copolymer is cross-linked by ionic terminal groups in the graft copolymer.

27. A pressure sensitive adhesive assembly comprising a body member having applied to at least one surface thereof an adhesive of any one of the preceding claims.

28. An assembly of Claim 27 in the form of a splicing tape for papermaking, printing or off-machine coating.

29. An assembly of Claim 28 in the form of an adhesive sheet or tape.

30. An assembly of Claim 28 in the form of an adhesive label.

31. An assembly of any one of Claims 27 to 30 wherein said body member comprises a water-dispersible backing layer.

## Patentansprüche

1. Haftklebstoff, der aus wasserlöslichem oder wasserdispergierbarem, normalerweise klebrigem Pfropfcopolymer zusammengesetzt ist, das aus einem oder mehreren wasserlöslichen Basismonomeren A, die Vinylmonomer umfassen, das hydrophiles Polymer bilden kann und eine T_{g} < 20°C aufweisen, wasserlöslichem oder wasserdispergierbarem Makromer C, das polymere Seitenketten des Pfropfcopolymers bildet, und gegebenenfalls einem oder mehreren mit dem A-Monomer copolymerisierten B-Monomeren zusammengesetzt ist, die hydrophiles oder hydrophobes Polymer bilden können, mit den Maßgaben, daß: wenn das C-Makromer in einer Menge von mindestens 45 Gew.% vorhanden ist, dann mindestens 5 Gew.% B-Monomer mit einer T_{g} > 20°C vorhanden sind, wenn das C-Makromer in einer Menge von 35 Gew.% oder weniger vorhanden ist, dann mindestens 5 Gew.% B-Monomer mit einer T_{g} < 0°C vorhanden sind, und jegliches vorhandene hydrophobe B-Monomer in einer Menge von 25 Gew.% oder weniger vorhanden ist, wobei sich jede der Mengen auf das Gesamtgewicht der Komponenten A, B und C bezieht, wobei der Klebstoff einen klebrigmacher- oder weichmacherfreien Polykentest-Klebwert von mindestens 300 g/cm², bestimmt gemäß ASTM D2979, einen Schäladhäsionswert von mindestens 220 g/cm (20 oz/in), bestimmt mittels PSTC 1, und einen Scnerungshaltewert von größer als 5 Minuten bei 500 g gemäß PSTC 7 aufweist.

2. Klebstoff nach Anspruch 1, bei dem das A-Monomer in einer Menge von 25 bis 70 Gew.% vorhanden ist, das B-Monomer in einer Menge von 0 bis 40 Gew.% vorhanden ist und das C-Makromer in einer Menge von 10 bis 65 Gew.% vorhanden ist, bezogen auf das Gesamtgewicht der Komponenten A, B und C.

3. Klebstoff nach Anspruch 2, bei dem das C-Makromer in einer Menge von mindestens 35 Gew.% vorhanden ist.

4. Klebstoff nach einem der vorhergehenden Ansprüche, bei dem hydrophobes B-Monomer in einer Menge bis zu 15 Gew.% vorhanden ist, bezogen auf das Gesamtgewicht der Komponenten A, B und C.

5. Klebstoff nach einem der vorhergehenden Ansprüche, bei dem das Gewichtsverhältnis von C-Makromer zu Monomeren A und B, die in dem Klebstoff vorhanden sind, im Bereich von 35:65 bis 65:35 liegt.

6. Klebstoff nach Anspruch 5, bei dem das Gewichtsverhältnis von C-Makromer zu Monomeren A und B, die in dem Klebstoff vorhanden sind, im Bereich von 40:60 bis 50:50 liegt.

7. Klebstoff nach einem der vorhergehenden Ansprüche, bei dem das A-Monomer ausgewählt ist aus Hydroxy(C₁- bis C₅-)alkylacrylaten, Hydroxy(C₁- bis C₅-)methalkylacrylaten, Dihydroxy(C₁- bis C₅-)alkylacrylaten, Dihydroxy(C₁- bis C₅-)alkylmethacrylaten und Mischungen derselben.

8. Klebstoff nach einem der vorhergehenden Ansprüche, bei dem das B-Monomer eine T_{g} > 20°C hat und hydrophil ist.

9. Klebstoff nach Anspruch 8, bei dem das B-Monomer wasserlösliches Vinylmonomer mit mindestens einem Stickstoffatom ist.

10. Klebstoff nach Anspruch 9, bei dem das B-Monomer ausgewählt ist aus N-monosubstituierten Acrylamiden, N,N-disubstituierten Acrylamiden und Mischungen derselben.

11. Klebstoff nach einem der vorhergehenden Ansprüche, der B-Monomer umfaßt, das Vinylmonomer ausgewählt aus Acryl- und Methacrylsäuren, Methoxyethylacrylat oder -methacrylat, Ethoxyethylacrylat oder -methacrylat, Methylacrylat oder -methacrylat, Ethylacrylat oder -methacrylat, Propylacrylat oder -methacrylat, n-Butylacrylat oder -methacrylat, Glycerinacrylat oder -methacrylat, Hydroxyethylmethacrylat, Hydroxypropylmethacrylat, Vinylpyrrolidon, Vinylcaprolactam und Mischungen derselben ist.

12. Klebstoff nach einem der vorhergehenden Ansprüche, das B-Monomer umfaßt, das monomeren Acryl- oder Methacrylsäureester von nicht-tertiärem Alkohol mit durchschnittlich 4 bis 12 Kohlenstoffatomen umfaßt.

13. Klebstoff nach einem der vorhergehenden Ansprüche, bei dem das Makromer durch die Formel X-(Y)ₚ-Z-R definiert ist, in der X ein mit Monomeren A und B copolymerisierbarer oder zur Bindung an copolymerisierte Monomere A und B fähiger Anteil ist, Y eine zweiwertige Verbindungsgruppe ist, Z ein wasserlöslicher oder wasserdispergierbarer Homo- oder Copolymeranteil ist, R eine endständige Gruppe ist und p 0 oder 1 ist.

14. Klebstoff nach Anspruch 13, bei dem X ein (Meth)acrylatanteil ist.

15. Klebstoff nach Anspruch 13 oder Anspruch 14, bei dem Z ausgewählt ist aus einem Polyalkylenoxidrest, Polyethyloxazolinrest, Polyacrylsäurerest, Polyvinylalkoholrest, Polyvinylpyrrolidonrest, Polyvinylcaprolactamrest und Polymethylvinyletherrest.

16. Klebstoff nach Anspruch 15, bei dem das Makromer durch die Formel
X-(Y)ₚ-(O-CₘH₂ₘ)ₙ-R
definiert ist, in der X ein mit Monomeren A und B copolymerisierbarer oder zur Bindung an copolymerisierte Monomere A und B fähiger Anteil ist, Y eine zweiwertige Verbindungsgruppe ist, R eine endständige Gruppe ist, m eine ganze Zahl von 2 bis 6 ist, n eine ganze Zahl von 5 bis 300 ist, und p 0 oder 1 ist.

17. Klebstoff nach Anspruch 16, bei dem das Makromer durch die Formel 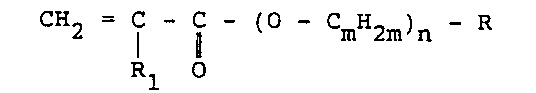 definiert ist, in der R₁ Wasserstoff oder C₁- bis C₅-Alkyl ist und R eine endständige Gruppe ist.

18. Klebstoff nach Anspruch 15, bei dem m 2 oder 3 ist und n eine ganze Zahl von 5 bis 30 ist.

19. Klebstoff nach einem der Ansprüche 13 bis 18, bei dem R OH oder C₁- bis C₅-Alkyl ist.

20. Klebstoff nach Anspruch 18 oder Anspruch 19, bei dem das Makromer ausgewählt ist aus ethoxylierten Hydroxyethyl(meth)acrylat und ethoxyliertem Hydroxypropyl(meth)acrylat.

21. Klebstoff nach Anspruch 15, bei dem das Makromer ausgewählt ist aus ethoxyliertem oder propoxyliertem Hydroxy(C₁- bis C₅-)alkyl(C₁- bis C₅-alk)acrylat und Polymethylvinylethermono(meth)acrylat.

22. Klebstoff nach Anspruch 13, bei dem das Copolymer mit Acrylamid, Butylacrylat, Vinylpyrrolidon und Acrylsäure als B-Monomere copolymerisiertes Hydroxy(C₁- bis C₅-)alkylacrylat als A-Monomer und wasserlösliches oder wasserdispergierbares Makromer umfaßt.

23. Klebstoff nach einem der Ansprüche 1 bis 22, der Vernetzungsmittel einschließt.

24. Klebstoff nach Anspruch 23, bei dem das Vernetzungsmittel dem vorgebildeten Pfropfcopolymer zugesetzt ist.

25. Klebstoff nach Anspruch 23 oder Anspruch 24, bei dem das Vernetzungsmittel eine Aziridinverbindung ist.

26. Klebstoff nach Anspruch 23, bei dem das Pfropfcopolymer über ionische endständige Gruppen in dem Pfropfcopolymer vernetzt ist.

27. Haftklebstoffzusammenstellung, die ein Körperteil umfaßt, bei dem auf mindestens einer Oberfläche Klebstoff gemäß einem der vorhergehenden Ansprüche aufgebracht ist.

28. Zusammenstellung nach Anspruch 27 in Form von Anklebband zur Papierherstellung, zum Drucken oder zum Separatstreichen.

29. Zusammenstellung nach Anspruch 28 in Form von Klebefolie oder Klebeband.

30. Zusammenstellung nach Anspruch 28 in Form eines Klebeetiketts.

31. Zusammenstellung nach einem der Ansprüche 27 bis 30, bei der das Körperteil eine wasserdispergierbare Rückseitenschicht umfaßt.

## Revendications

1. Adhésif sensible à la pression, composé d'un copolymère greffé normalement collant hydrosoluble ou dispersable dans l'eau constitué d'un ou plusieurs monomères de base hydrosolubles A comprenant un monomère vinylique capable de former un polymère hydrophile et ayant une T_{g} < 20°C, d'un macromère hydrosoluble ou dispersable dans l'eau C formant les chaînes latérales polymériques dudit copolymère greffé, et, éventuellement, copolymérisés avec ledit monomère A, d'un ou plusieurs monomères B capables de former un polymère hydrophile ou hydrophobe, avec les clauses conditionnelles suivantes : quand ledit macromère C est présent en une quantité d'au moins 45% en poids, alors au moins 5% en poids d'un monomère B ayant une T_{g} > 20°C doit être présent, quand ledit macromère C est présent en une quantité de 35% en poids ou moins, alors au moins 5% en poids d'un monomère B ayant une T_{g} < 0°C doit être présent, et tout monomère B hydrophobe présent est présent en une quantité de 25% en poids ou moins, chacune desdites quantités étant rapportée au poids total des constituants A, B et C, ledit adhésif présentant une valeur de collant après essai avec une sonde Polyken dépourvue d'agent donnant du collant ou de plastifiant d'au moins 300 g/cm² telle que déterminée par ASTM D2979, une valeur d'adhésion après essai d'arrachement d'au moins 220 g/cm (20 once/pouce) telle que déterminée par PSTC 1, et une valeur de tenue au cisaillement supérieure à 5 minutes telle que déterminée à 500 grammes suivant PSTC 7.

2. Adhésif selon la revendication 1, dans lequel ledit monomère A est présent en une quantité de 25 à 70% en poids, ledit monomère B est présent en une quantité de 0 à 40% en poids, et ledit macromère C est présent en une quantité de 10 à 65% en poids, rapporté au poids total des constituants A, B et C.

3. Adhésif selon la revendication 2, dans lequel ledit macromère C est présent en une quantité d'au moins 35% en poids.

4. Adhésif selon l'une quelconque des revendications précédentes, dans lequel un monomère B hydrophobe est présent en une quantité pouvant s'élever jusqu'à 15% en poids, rapporté au poids total des constituants A, B et C.

5. Adhésif selon l'une quelconque des revendications précédentes, dans lequel le rapport pondéral du macromère C aux monomères A et B présents dans ledit adhésif varie de 35:65 à 65:35.

6. Adhésif selon la revendication 5, dans lequel le rapport pondéral du macromère C aux monomères A et B présents dans ledit adhésif varie de 40:60 à 50:50.

7. Adhésif selon l'une quelconque des revendications précédentes, dans lequel ledit monomère A est choisi parmi les acrylates d'hydroxy(alkyle en C₁ à C₅), les méthacrylates d'hydroxy(alkyle en C₁ à C₅), les acrylates de dihydroxy(alkyle en C₁ à C₅), les méthacrylates de dihydroxy(alkyle en C₁ à C₅), et les mélanges de ceux-ci.

8. Adhésif selon l'une quelconque des revendications précédentes, dans lequel ledit monomère B qui a une T_{g} > 20°C est hydrophile.

9. Adhésif selon la revendication 8, dans lequel ledit monomère B est un monomère vinylique hydrosoluble possédant au moins un atome d'azote.

10. Adhésif selon la revendication 9, dans lequel ledit monomère B est choisi parmi les acrylamides N-monosubstitués; les acrylamides N,N-disubstitués et les mélanges de ceux-ci.

11. Adhésif selon l'une quelconque des revendications précédentes, comprenant un monomère B qui est un monomère vinylique choisi parmi les acides acrylique et méthacrylique, l'acrylate ou le méthacrylate de méthoxyéthyle, l'acrylate ou le méthacrylate d'éthoxyéthyle, l'acrylate ou le méthacrylate de méthyle, l'acrylate ou le méthacrylate d'éthyle, l'acrylate ou le méthacrylate de propyle, l'acrylate ou le méthacrylate de n-butyle, l'acrylate ou le méthacrylate de glycérol, le méthacrylate d'hydroxyéthyle, le méthacrylate d'hydroxypropyle, la vinylpyrrolidone, le vinylcaprolactame et les mélanges de ceux-ci.

12. Adhésif selon l'une quelconque des revendications précédentes, comprenant un monomère B qui comprend un ester d'acide acrylique ou méthacrylique monomérique d'un alcool non tertiaire ayant de 4 à 12 atomes de carbone en moyenne.

13. Adhésif selon l'une quelconque des revendications précédentes, dans lequel ledit macromère est défini par la formule X-(Y)ₚ-Z-R, dans laquelle X est un fragment copolymérisable avec les monomères A et B ou capable de se fixer aux monomères A et B copolymérisés, Y est un groupe de liaison divalent, Z est un fragment homo- ou copolymérique hydrosoluble ou dispersable dans l'eau, R est un groupe terminal, et p vaut 0 ou 1.

14. Adhésif selon la revendication 13, dans lequel X est un fragment (méth)acrylate.

15. Adhésif selon la revendication 13 ou de la revendication 14, dans lequel Z est choisi parmi un radical poly(oxyde d'alkylène), un radical poly(éthyloxazoline), un radical poly(acide acrylique), un radical poly(alcool vinylique), un radical poly(vinylpyrrolidone), un radical poly(vinylcaprolactame) et un radical poly(méthylvinyléther).

16. Adhésif selon la revendication 15, dans lequel ledit macromére est défini par la formule :
X-(Y)ₚ-(O-CₘH₂ₘ)ₙ-R
dans laquelle X est un fragment copolymérisable avec les monomères A et B ou capable de se fixer aux monomères A et B copolymérisés, Y est un groupe de liaison divalent, R est un groupe terminal, m est un nombre entier de 2 à 6, n est un nombre entier de 5 à 300, et p vaut 0 ou 1.

17. Adhésif selon la revendication 16, dans lequel le macromère est défini par la formule
CH₂ = C(R₁)-C(=O)-(O-CₘH₂ₘ)ₙ-R
dans laquelle R₁ est un atome d'hydrogène ou un groupe alkyle en C₁ à C₅ et R est un groupe terminal.

18. Adhésif selon la revendication 15, dans lequel m vaut 2 ou 3 et n est un nombre entier de 5 à 30.

19. Adhésif selon l'une quelconque des revendications 13 à 18, dans lequel R est OH ou un groupe alkyle en C₁ à C₅.

20. Adhésif selon la revendication 18 ou de la revendication 19, dans lequel ledit macromère est choisi parmi le (méth)acrylate d'hydroxyéthyle éthoxylé et le (méth)acrylate d'hydroxypropyle éthoxylé.

21. Adhésif selon la revendication 15, dans lequel ledit macromère est choisi parmi l'(alk- en C₁ à C₅)acrylate d'hydroxy(alkyle en C₁ à C₅) éthoxylé ou propoxylé et le mono(méth)acrylate de polyméthylvinyléther.

22. Adhésif selon la revendication 13, dans lequel ledit copolymère comprend un acrylate d'hydroxy(alkyle en C₁ à C₅) en tant que monomère A copolymérisé avec l'acrylamide, l'acrylate de butyle, la vinylpyrrolidone et l'acide acrylique en tant que monomères B, et un macromère hydrosoluble ou dispersable dans l'eau.

23. Adhésif selon l'une quelconque des revendications 1 à 22, qui renferme un agent réticulant.

24. Adhésif selon la revendication 23, dans lequel l'agent réticulant est ajouté au copolymère greffé préformé.

25. Adhésif selon la revendication 23 ou de la revendication 24, dans lequel l'agent réticulant est un composé d'aziridine.

26. Adhésif selon la revendication 23, dans lequel le copolymère greffé est réticulé par des groupes terminaux ioniques dans le copolymère greffé.

27. Dispositif adhésif sensible à la pression comprenant un élément de corps dont au moins une face est revêtue d'un adhésif selon l'une quelconque des revendications précédentes.

28. Dispositif selon la revendication 27 sous la forme d'un ruban adhésif pour raccord pour la fabrication du papier, l'impression ou le couchage hors machine.

29. Dispositif selon la revendication 28 sous la forme d'une feuille ou d'une bande adhésive.

30. Dispositif selon la revendication 28 sous la forme d'une étiquette auto-adhésive.

31. Dispositif selon l'une quelconque des revendications 27 à 30 dans lequel ledit élément de corps comprend une couche de renfort dispersable dans l'eau.
